# EUROPEAN PATENT APPLICATION

(11) **EP 0 609 167 A1**
(43) Date of publication of application: **03.08.1994**
(21) Application number: 94500009.9
(22) Date of filing: 26.01.1994
(51) Int. Cl.: A61M 5/32, A61M 5/50

(54) **Self-destructing retractable syringe**

(30) Priority: 27.01.1993 ES 9300143
(71) Applicant: Montesinos Mulleras, Vincente, E-28015 Madrid (ES)
(72) Inventor: Montesinos Mulleras, Vincente, E-28015 Madrid (ES)
(74) Representative: Garcia Cabrerizo, Francisco

(57) **Abstract**

The syringe includes a casing (1), a plunger (2) which moves inside of it and a needle-bearer (5) with the respective needle (3) coupled to the end of the casing. The plunger is hollow and the surface (10) which closes its pushing end has an attenuated annular line (11) and a concentric projection (12) with a cutting edge, while the plate (7) which is part of the needle-bearer, which is located in the bottom of the casing, has an attenuating line (9) and an annular cutting projection (8) complementing the projection and the attenuation, respectively, of the closing of the plunger; at the end of the travel of the plunger trajectory, the cut through said weakening lines takes place by means of a spring (4), the divided disks and the needle itself being pushed inside the plunger. Thus, the syringe is disabled and conceals the needle after being used.

## Description

The invention refers to a self-destructible retractile syringe, which has been designed and structured in a very simple manner without detriment to a great efficacy, since the syringe in question fulfills perfectly and in a safe manner the condition of disablement which follows its first and only use.

At present, the use of disposable syringes, whose obvious purpose is that of attempting to avoid contagion among patients, is practically universal. Likewise, there is a tendency for the needle of the syringe to remain concealed in some manner after use, to avoid puncturing, health personnel and therefore contagion.

There are numerous types of syringes designed for those purposes, although the marketing of such syringes is in many cases reduced by the structural complexity they offer, while in other cases, marketing is likewise reduced because although the syringes may be structurally simple, they are not very reliable or effective for the intended purposes.

The syringe which is recommended has been conceived to resolve all these problems to full satisfaction; the problems of efficacy and safety are resolved on the basis of a simple solution which will allow the marketing of said syringe without problems of any type.

Structurally, it is composed, as is conventional, starting from a cylindrical casing within which the correspoding plunger may be moved for the suction and/or pushing of the injectable liquid, complemented by the corresponding hypodermic needle which may be connected to the anterior end of said casing.

Starting from these conventional characteristics, the syringe of the invention presents the following particularities:
The plunger is hollow, and the surface which makes up the closed pushing end is flat and is affected by a weakening line and by a concentric crown with a cutting projecting edge.

The casing has an axial and external neck in the needle-coupling end with a first section of greater diameter than a second section, the latter having a spring mounted in it which is retained toward the outside by the corresponding step and toward the inside by the needle-coupling means.

The needle-coupling means is made up of a disk or plate which on one face has a neck for coupling with the needle, a neck which remains located in the first section or that of greatest diameter of the axial neck of the casing, while the plate or disk is located and fixed in the bottom of the casing itself, this plate or disk being affected in its face facing the end of the plunger by a concentric and projecting crown and an attenuating line, concentric and complementary, respectively, to the attenuating line and crown of said end of the plunger.

According to this structuring and since the height of the crown at the end of the plunger is slightly greater than that provided in the needle-bearing plate, when the injection of the liquid is produced by sliding the plunger toward the output end, the crown at the end of the plunger cuts the needle-bearing plate on the attenuating line and afterwards the cut on the end of the plunger is performed by the crown of the plate, with which the spring provided in the outer and axial neck of the casing pushes the divided parts toward the inside of the plunger, disabling the latter and therefore the syringe, besides brings about the concealment of the needle inside said plunger.

To facilitate a better understanding of the characteristics of the invention, a detailed description will be given, based on a set of drawings which it attached to this descriptive report, forming an integral part of the same and in which the following has been represented with a merely illustrative and nonlimiting nature:
In Figure 1, a longitudinal cross section of the syringe which is the subject of the invention is shown.
In Figure 2, a perspective of the needle-bearing means or element is shown; that is to say, the plate with its neck, cutting crown and attenuated concentric line, with the needle attached.

According to and as can be seen in, these figures, the syringe of the invention includes a cylindrical casing (1), in whose interior the corresponding plunger (2) may be moved for drawing and/or pushing the extractable and/or injectable liquid, respectively. Said casing (1) includes on its anterior or output end a concentric and axial neck (3), which inside has two sections of different diameter, one for placing a spring (4) and the other, which corresponds to the one with greater diameter, for the positioning of the neck (5) belonging to the needle-bearing element, said needle (6) existing to the outside through the end of said neck.

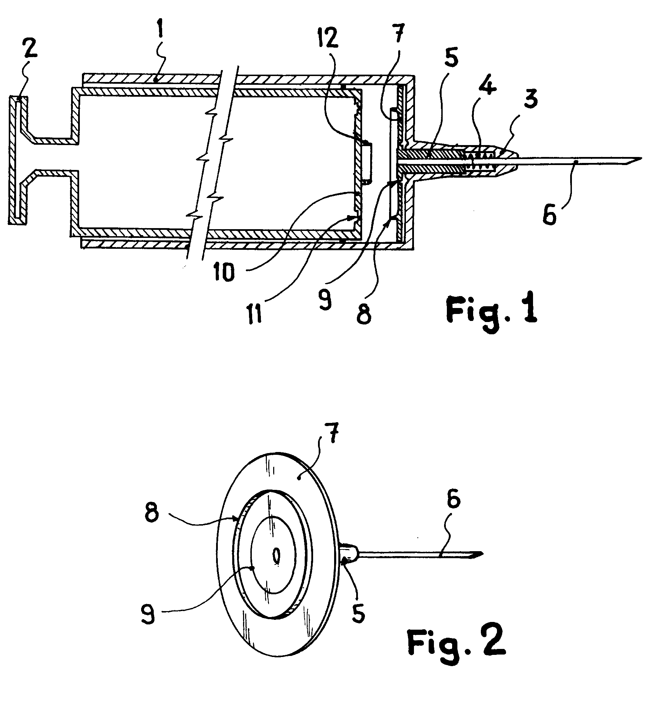

The spring (4) is held between a step provided before the output end and the end itself of the neck (5) belonging to the needle-bearer, the latter being complemented by a circular plate (7) which is held in the bottom of the casing (1), as is seen in Figure 1.

This plate (7) of the needle-bearer is affected an annular attenuating outline or line (9) and by a projection in the shape of a circular crown (8), of greater diameter than the attenuating circumference (9) and affected by a cutting edge.

Said crown (8) with a cutting edge faces the closing surface (10) of the plunger (2), the latter being hollow. This closing surface (10) is affected by an attenuating line or outline (11) whose diameter corresponds to that of the crown (8) of the plate. Said closing surface (10) also has on its face facing the plate (7) a circular crown (12), also formed by an annular projection with a cutting edge, the diameter of this crown (12) being equal to that of the attenuating line (9) of the plate (7).

The height of the crown (12) is slightly greater than the height of the crown (8).

The functioning is as follows:
The drawing and corresponding emptying of the liquid being performed, as is conventional, when the closed pushing end of the plunger (2) reaches the area next to the bottom of the casing (1), i.e., plate (7) of the needle-bearer, the cutting edge of the crown (12) of the closed bottom surface (10) of the plunger will attack the attenuating line (9) of plate (7), producing the division of the latter. In this manner, the disk divided from this plate (7) will remain free, being pushed by the spring (4) against the closing surface itself (10) of the plunger (2), the attack of crown (8) on the attenuating line (11) of said closing surface (10) then taking place, with which the correspondingly divided disk shall be pushed by the force of the coil or spring (4) toward the inside of the plunger (2), the latter remaining disabled and the needle simultaneously also remaining lodged in that plunger, avoiding all danger of contagion and/or injury.

## Claims

1. Self-destructible retractile syringe composed of a cylindrical casing within which the corresponding plunger for drawing and/or pushing the extraction and/or injection liquid is moved, the end of the casing having an axial neck for the passage of the respective hypodermic needle and for positioning the needle-bearing element, essentially characterized in that the plunger is hollow and the circular surface which closes its pushing end has an annular attenuating line and a projection which is also annular, the latter determining a crown with a cutting edge; while the needle-bearing element has a circular plate located at the bottom of the casing, which plate has in its face facing the closing surface of the plunger an annular projection which determines a crown with a cutting edge, and an attenuating line which is also annular, with the particularity that the crown of said plate is of the same diameter as the attenuated circumference of the closing surface of the plunger, while the crown of the latter is of the same diameter as the circumference of the attenuating line of said plate.

2. Self-destructible retractile syringe according to Claim 1, characterized in that a concentric neck housed in a first section of the axial and outer neck of the casing emerges from the plate of the needle-bearing element, while in the inner section of lesser diameter of said axial neck a pushing spring is positioned which rests on an internal step of said neck and in the end itself of the neck belonging to the needle-bearer, said spring pushing the disks freed in the division along the attenuating lines of the plate and the closing surface of the plunger, moving said disks and the needle itself toward the inside of the plunger.

3. Self-destructible retractile syringe according to the previous claims, characterized in that the height of the projection forming the crown corresponding to the closing surface of the plunger is slightly larger than that of the projection determining the crown corresponding to the plate of the needle-bearing element.
